# EUROPÄISCHE PATENTSCHRIFT

(11) **EP 0 685 980 B1**
(45) Veröffentlichungstag und Bekanntmachung des Hinweises auf die Patenterteilung: **02.09.1998**
(21) Anmeldenummer: 95107542.3
(22) Anmeldetag: 18.05.1995
(51) Int. Cl.: H04R 9/02, H04R 7/16

(54) **Lautsprecher**
Loudspeaker
Haut-parleur

(30) Priorität: 01.06.1994 DE 4419249
(43) Veröffentlichungstag der Anmeldung: 06.12.1995
(73) Patentinhaber: NOKIA TECHNOLOGY GmbH, 75175 Pforzheim (DE)
(72) Erfinder: Geisenberger, Stefan, D-94315 Straubing (DE); Aigner, Manfred, D-94374 Schwarzach (DE)

(56) Entgegenhaltungen:
- EP-A- 0 270 981
- EP-A- 0 690 658
- DE-A- 4 419 312
- FR-A- 2 186 079
- FR-A- 2 244 094
- FR-A- 2 668 018
- GB-A- 2 125 491
- GB-A- 2 186 761
- JP-A-54 072 031

## Beschreibung

### Technisches Gebiet

Die Erfindung befaßt sich mit der Verbindung von Lautsprechermembran und Schwingspulenträger.

### Stand der Technik

Gemäß dem Stand der Technik wird die Lautsprechermembran mit dem rohrförmig ausgebildeten Schwingspulenträger allgemein mit Klebstoff verbunden. Diese Technik hat sich bei Lautsprechern bewährt, welche bis etwa 120 Grad Celsius temperaturbelastet werden. Sollen Lautsprecher jedoch bei höheren Betriebstemeperaturen als der eben angegebenen zuverlässig betrieben werden, sind die eben genannten Klebeverbindungen nicht mehr einsetzbar. Auch der Einsatz von höher wärmebelastbaren Klebstoffen können das Festigkeitsproblem der mechanisch stark belasteten Verbindungen von Schwingspulenträger und Lautsprechermembran nur unzureichend lösen, da auch diese Klebstoffe nicht geeignet sind, oberhalb von 200 Grad Celsius eingesetzt zu werden. Außerdem sind derart verbesserte -aber mitunter auch toxische-Klebstoffe in ihrer Herstellung und Verarbeitung aus Gründen des Umweltschutzes in heutiger Zeit nicht mehr zu rechtfertigen.

Aus der EP-A-0 270 981 ist ein Lautsprecher gemäß dem Oberbegriff des Anspruchs 1 bekannt.

Einstückige Ausbildungen von Lautsprechermembran und Schwingspulenträger sind zwar auch bekannt, können aber in den für diese Kombination geforderten Gewichtsverhältnissen nur aus Kunststoff oder Papier/Pappe gebildet werden. Einstückige Ausbildungen aus Leichtmetall, welche gegenüber den Kunstoff- oder Papierausbildungen auch höheren Betriebstemperaturen zugänglich sind, sind derzeit nicht verfügbar. Versuche der Anmelderin, einstückige Ausbildungen von Lautsprechermembran und Schwingspulenträger etwa aus Aluminium zu bilden, haben gezeigt, daß sich in Tiefziehprozessen der Hals (welcher als Schwingspulenträger dient) mit einer für den Membrankegel notwendigen Wandstärke von etwa 200 um nur bis zu einer Länge von etwa 10mm herstellen läßt. Derartige Halslängen ist aber nicht geeignet als Schwingspulenträger zu dienen. Außerdem haben Wandstärken des Schwingspulenträgers von 200 um große Luftspaltweiten zur Folge. Dies verringert die Luftspaltinduktion und wirkt sich negativ auf die Temperaturstabilität des Magnetsystems aus.

Daher liegt der Erfindung die Aufgabe zugrunde, eine Verbindung von Lautsprechermembran und Schwingspulenträger anzugeben, welche einfach herstellbar ist und welche bis über 400 Grad Celsius temperaturbeständig ist.

### Darstellung der Erfindung

Diese Aufgabe wird durch die Merkmale von Anspruch 1 gelöst. Vorteilhafte Aus- und Weiterbildungen sind den Ansprüchen 2 und 3 entnehmbar.

Prinzip von Anspruch 1 ist der Gegenstand, die Verwendung von Klebstoffen bei der Verbindung von Schwingspulenträger und Lautsprechermembran auszuschließen und stattdessen beide eben genannten Lautsprecherteile rein mechanisch miteinander zu verbinden.

Gemäß Anspruch 1 wird dazu angegeben, auch die Lautsprechermembran aus Metall zu bilden und mit einem Hals zu versehen, der in den von der konischen Membran gebildeten Raum ragt, sowie die Verbindung von Hals und oberem Rand des Schwingspulenträgers als Faltverbindung auszuführen. Diese Falt- bzw. Bördelverbindung gestattet es, die Lautsprechermembran und den Hals als Tiefziehteil herzustellen, da die im Tiefziehprozeß herstellbaren Halslängen ausreichen, um eine Faltverbindung zwischen dem Schwingspulenträger und dem Hals der Lautsprechermembran auszuführen.

Ist gemäß Anspruch 2 der Übergangsbereich von Lautsprechermembran zum Hals als Radius ausgebildet, muß ein Abbrechen des Halses während des Tiefziehprozesses nicht befürchtet werden.

Sind gemäß Anspruch 3 die oberen Rände von Schwingspulenträger und Hals mit in Richtung zur Lautsprecherachse verlaufenden Einschnitten versehen, ist die Herstellbarkeit der Falt- bzw. Börgelverbindung weiter vereinfacht.

### Kurze Darstellung der Figuren

Es zeigen:
- Figur 1: einen Schnitt durch Lautsprechermembran und Schwingspulenträger;
- Figur 2a-c: eine weiter Darstellung gemäß Figur 1 in drei Fertigungsstufen; und

### Wege zum Ausführen der Erfindung

Die Erfindung soll nun anhand der Figuren näher erläutert werden.

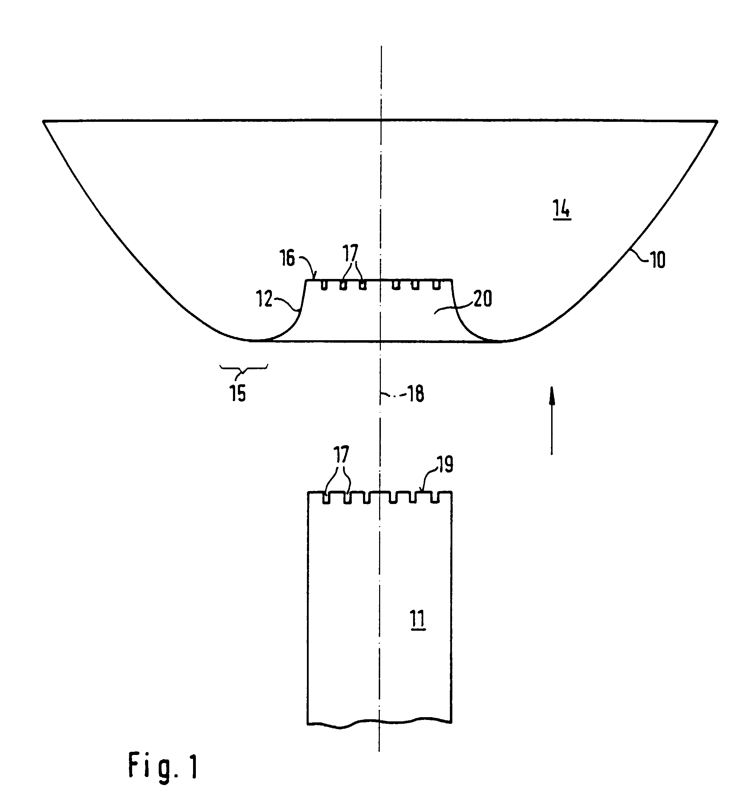

Die schematische Schnittdarstellung gemäß Figur 1 zeigt im oberen Teil eine Lautsprechermembran 10 und im unteren Teil einen Schwingspulenträger 11. Beide Teile 10, 11 sind im hier gezeigten Ausführungsbeispiel aus Aluminium gebildet. Dies heißt nicht, daß die Lautsprechermembran 10, der Schwingspulenträger 11 oder auch beide Teile 10, 11 nicht aus einem anderen Metall gebildet sein können.

Die in etwa konisch geformte Lautsprechermembran 10 ist mit einem angeformten Hals 12 versehen. Dieser Hals 12 ragt in den vom Innenmantel der konischen Membran 10 begrenzten Raum 14 hinein. Der Übergangsbereich 15 von Lautsprechermembran 10 und Hals 12 ist gerundet ausgebildet. Der obere Rand 16 des Halses 12 ist mit Einschnitten 17 versehen, um den später noch näher erörterten Faltprozess zu erleichtern. Diese Einschnitte 17 verlaufen parallel zur Lautsprecherachse 18.

Der Schwingspulenträger 11 ist rohrförmig gestaltet und hat an seinem oberen Rand 19 ebenfalls parallel zur Lautsprecherachse 18 verlaufende Einschnitte 17. Auch diese Einschnitte 17 im oberen Rand 19 des Schwingspulenträgers 11 dienen zur Verbesserung des noch später zu erörternden Faltprozesses.

Um eine Einheit aus Lautsprechermembran 10 und Schwingspulenträger 11 zu bilden, wird beispielsweise der Schwingspulenträger 11 in Pfeilrichtung in die Halsöffnung 20 der Lautsprechermembran 10 eingeschoben.

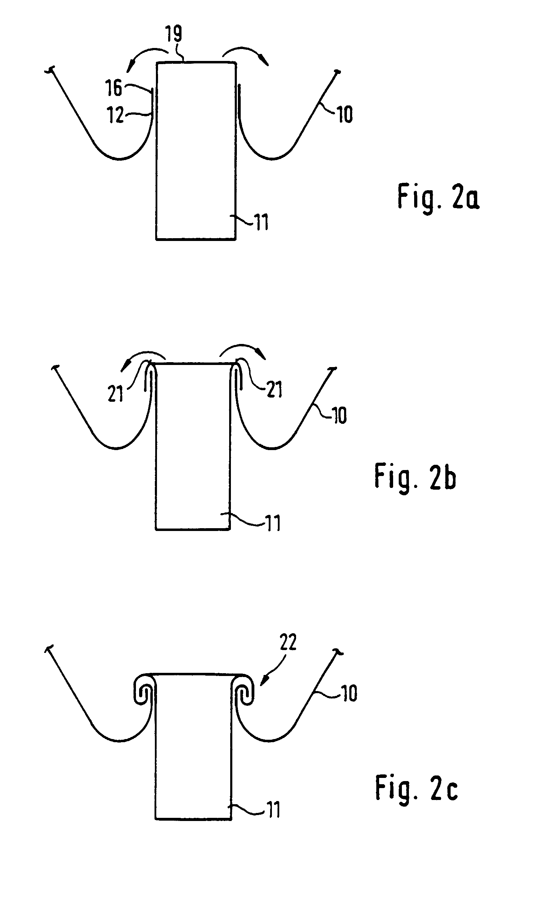

Dieser Zustand ist in Figur 2a schematisch dargestellt. Deutlich ist der Darstellung entnehmbar, daß, wenn der Schwingspulenträger 11 seine Endlage in der Halsöffnung 20 eingenommen hat, der obere Rand 19 des Schwingspulenträgers 11 den oberen Rand 16 des Halses 12 überragt. In diesem Zusammenhang sei darauf hingewiesen, daß bei der Darstellung gemäß der Figuren 2a-c auf die Sichtbarmachung der in Figur 1 gezeigten Einschnitte 17 verzichtet wurde.

Um die Lautsprechermembran 10 mit dem Schwingspulenträger 11 zu verbinden, wird der obere Rand 19 des Schwingspulenträgers 11 nach außen um den oberen Rand 16 des Halses 12 gebogen (dargestellt durch die gebogenen Pfeile in Figur 2a).

Ist dieser Faltvorgang abgschlossen, hat die Schwingspulenträger-Lautsprechermembran-Kombination ein Aussehen, welches in Figur 2b gezeigt ist. Um die in Zusammenhang mit Figur 2a erörterte erste Faltung sichtbar zu machen, wurde in der Darstellung gemäß Figur 2b darauf verzichtet, ein enges Aneinanderliegen der Ränder 16, 19 zu zeigen.

Ist der Zustand gemäß Figur 2b erreicht, wird der durch die erste Faltung gebildete obere Rand 21 nochmals nach außen umgebogen (angedeutet durch die in Figur 2b gezeigten Pfeile).

Ist die zweite Faltung abgeschlossen, stellt sich ein Zustand ein, der in Figur 2c gezeigt ist. Die Faltverbindung 22 verbindet den Spulenträger 11 und die Lautsprechermembran 10 fest miteinander, da nach der zweiten Faltung die umgebogenen Randbereiche abstandslos übereinander liegen. Letzteres wurde in Figur 2c nicht gezeigt, um den Verlauf der Faltung besser zu verdeutlichen.

Soll bei der in Figur 2c gezeigten Faltverbindung 22 die Haftreibungskraft der miteinander in Berührung stehenden Bereiche von Hals 12 und Schwingspulenträger 11 bei steigenden Betriebstemperaturen erhöht werden, sollte dasjenige Bauteil, welches nur einmal gefaltet wird (in Figur 2a-c der Hals 12), einen größeren Wärmeausdehnungskoeffizienten haben als das Bauteil, welches mehrfach umgebogen wird (hier der obere Rand des Schwingspulenträgers 11).

## Patentansprüche

1. Lautsprecher
mit einer konisch geformten Lautsprechermembran (10) und mit einem Schwingspulenträger (11), dessen oberer Rand (19) mit der Lautsprechermembran (10) verbunden ist,
dadurch gekennzeichnet,
daß der Schwingspulenträger (11) und die Lautsprechermembran (10) aus Metall gebildet sind und daß die Lautsprechermembran (10) einen Hals (12) aufweist, der in den von der konisch geformten Lautsprechermembran (10) begrenzten Raum (14) ragt, und
daß die Verbindung von Hals (12) der Lautsprechermembran (10) und oberem Rand (19) des Schwingspulenträgers (11) als Faltverbindung (22) dieser beiden Elemente ausgeführt ist.

2. Lautsprecher nach Anspruch 1,
dadurch gekennzeichnet,
daß die oberen Ränder (19,16) von Schwingspulenträger (11) und Hals (12) der Lautsprechermembran (10) parallel zur Lautsprecherachse (18) verlaufende Einschnitte (17) aufweisen.

3. Lautsprecher nach Anspruch 1 oder 2,
dadurch gekennzeichnet,
daß der Übergangsbereich (15) von Lautsprechermembran (10) und Hals (11) gerundet ausgebildet ist.

## Claims

1. Loudspeaker
having a cone-shaped loudspeaker diaphragm (10) and having a voice coil former (11) whose upper rim (19) is joined to the loudspeaker diaphragm (10),
**characterised in that**
the voice coil former (11) and the loudspeaker diaphragm (10) are formed from metal, and that the loudspeaker diaphragm (10) has a neck (12), which projects into the space (14) bounded by the cone-shaped loudspeaker diaphragm (10), and that
the connection between the neck (12) of the loudspeaker diaphragm (10) and the upper rim (19) of the voice coil former (11) is constructed as a bellows connection (22) for these two elements.

2. Loudspeaker according to Claim 1,
**characterised in that**
the upper rims (19, 16) of the voice coil former (11) and the neck (12) of the loudspeaker diaphragm (10) have notches (17) running parallel to the loudspeaker axis (18).

3. Loudspeaker according to Claim 1 or 2,
**characterised in that**
the transition region (15) between loudspeaker diaphragm (10) and neck (11) is of a rounded construction.

## Revendications

1. Haut-parleur muni d'une membrane de haut-parleur (10) de forme conique et d'un support de bobine mobile (11), dont le bord supérieur (19) est relié à la membrane de haut-parleur (10), caractérisé en ce que le support de bobine mobile (11) et la membrane de haut-parleur (10) sont formés en métal et en ce que la membrane de haut-parleur (10) présente une collerette (12) qui s'étend dans l'espace (14) délimité par la membrane de haut-parleur (10) de forme conique, et en ce que la liaison de la collerette (12) de la membrane de haut-parleur (10) et du bord supérieur (19) du support de bobine mobile (11) est réalisé sous la forme d'un assemblage par pliage (22) de ces deux éléments.

2. Haut-parleur selon la revendication 1, caractérisé en ce que les bords supérieurs (19, 16) du support de bobine mobile (11) et la collerette (12) de la membrane de haut-parleur (10) présentent des entailles (17) parallèles à l'axe du haut-parleur (18).

3. Haut-parleur selon la revendication 1 ou 2, caractérisé en ce que la zone de transition (15) de la membrane de haut-parleur (10) et de la collerette (12) est conçue de façon arrondie.
